# EUROPEAN PATENT APPLICATION

(11) **EP 3 424 533 A1**
(43) Date of publication of application: **09.01.2019**
(21) Application number: 17305869.4
(22) Date of filing: 05.07.2017
(51) Int. Cl.: A61K 41/00

(54) **METHODS FOR TREATING TUMORS**

(71) Applicant: NH THERAGUIX, 69100 Villeurbanne (FR); Université Claude Bernard Lyon 1, 69100 Villeurbanne (FR); Centre National de la Recherche Scientifique (CNRS), 75016 Paris (FR); INSERM (Institut National de la Santé et de la Recherche Médicale), 75013 Paris (FR); Institut Gustave Roussy, 94805 Villejuif Cédex (FR)
(72) Inventor: LUX, François, 69006 LYON (FR); TILLEMENT, Olivier, 69270 Fontaines Saint Martin (FR); PERFETTINI, Jean-Luc, 77100 MEAUX (FR); DEUTSCH, Eric, 75011 PARIS (FR); LAW, Frédéric, 94290 VILLENEUVE LE ROI (FR); ALLOUCH, Awatef, 94360 BRY SUR MARNE (FR)
(74) Representative: Cabinet Plasseraud

(57) **Abstract**

The invention relates to methods for treating tumors. In particular, the invention provides novel use of nanoparticles in combination with ionizing radiations for treating tumors, wherein the combined effect of nanoparticles induces senescence and/or cannibalism of the tumor cells.

## Description

### Technical Domain

The invention relates to methods for treating tumors. In particular, the invention provides novel use of nanoparticles in combination with ionizing radiations for treating tumors, wherein the combined effect of nanoparticles induces senescence and/or cannibalism of the tumor cells.

### Background

The radiation therapy (also known as radiotherapy) is one of the most used anti-tumor strategies. More than half of all patients with cancer are treated with ionizing radiation (IR) alone or in combination with surgery or chemotherapy.¹ Recent progresses realized in medical physics (with the development of low/high energy radiation, the implementation of mono-, hypo- or hyper-fractionation schedule and the diversification of dose rates used) and the development of innovative medical technologies (such as the 3D-conformational radiotherapy (3D-CRT), the intensity modulated radiation therapy (IMRT), the stereotactic radiosurgery (SRS) and the functional imaging)) contribute to better deliver the efficient doses of radiation on tumors whilst sparing surrounding healthy tissues, which is the most usual side effect of radiation therapy.² Several applications of nanomedecine (such as radioisotope-labeled or metallic nanoparticles) have been developed to improve this therapeutic index by using nanomaterials as imaging or contrast agents to better deliver the radiation doses into tumor sites and/or as radiosensitizers, to enhance the dose deposition in tumors and reduce irradiation-related side-effects.^{3, 4, 5, 6, 7} Considering that the radiation dose absorbed by any tissues is related to the square of relative atomic number (Z²) of the material (where Z is the atomic number)⁸, nanoparticles containing high-Z atoms (such as gold or gadolinium) have been extensively investigated for their potential to improve radiotherapy. Under exposure to ionizing radiations, heavy-metal based nanoparticles produce photons and Auger electrons that improve the total dose rate deposition into the tumors, induce the production of reactive oxygen species (ROS) and cause cellular damages on many tumors (including for example melanoma, glioblastoma, breast and lung carcinomas).^{9, 10, 11} Despite the fact that several preclinical animal models revealed the ability of the combination of heavy-metal based nanoparticles with ionizing radiation to reduce tumor growth, there is still a need to improve the use of nanoparticles in anti-cancer treatments in combination with ionizing radiations.

In this the present disclosure, the inventors explored the ability of the combination of high-Z element containing nanoparticles, and in particular gadolinium-based nanoparticles (GdBN), with ionizing radiation to induce both cellular senescence and the death through non-cell-autonomous mechanisms. The inventors revealed that the irradiation of cancer cells in presence of GdBN enhances the ability of irradiated cancer cells to undergo senescence. In parallel, they observed that irradiated cancer cells also exhibit cannibalistic activity and eliminate after live cell engulfment, both irradiated and non-irradiated neighboring cancer cells. They further deciphered signaling pathways that are involved in the biological effects elicited by the irradiation of cancer cells in presence of GdBN and revealed that after an irradiation in presence of GdBN, the proliferation of cancer cells may be impaired by both senescence and cellular cannibalism inductions, said inductions being mediated by NOX5 and ROCK1 activity. These findings opened new insights for optimized anti-cancer treatments combining nanoparticles and ionizing radiations.

### Brief Description

A first aspect of the present disclosure relates to a method of treating a tumor in a subject in need thereof, the method comprising
a. administering an efficient amount of a suspension of nanoparticles to the tumor of a subject in need thereof, said nanoparticles comprising an element with an atomic Z number higher than 40, preferably higher than 50, and having a mean hydrodynamic diameter below 10 nm, preferably below 5 nm, for example between 1 and 5 nm, and,
b. exposing said tumor comprising the nanoparticles to an efficient dose of ionizing radiations,
wherein the combined effect of the ionizing radiations and the nanoparticles induce senescence and/or cellular cannibalism to the irradiated tumor cells.

In specific embodiments, said tumor is exposed to a dose per fraction of ionizing radiations of at least 3 Gy, and for example between 3 Gy and 9 Gy, or between 5 and 7 Gy. In a more specific embodiment of the previous embodiment, the total dose of ionizing radiations is administered in no more than 10 fractions, for example within 3 to 8 consecutive weeks.

In one specific embodiment, the method further includes a step of determining NOX5 and/or ROCK1 expression level or activity in the tumor, prior to the treatment step. Typically, the subject to be treated is selected among the subjects having a tumor wherein NOX5 and/or ROCK1 activity or expression level higher than or at least substantially the same as a control value.

In specific embodiments, the method further comprises a step of administering an enhancer agent of NOX5 and/or ROCK1 activity, prior to, or concomitantly, or after the exposure step to ionizing radiations, for increasing NOX5 and/or ROCK1 activity in the tumor.

In preferred embodiments, the combined effect of the ionizing radiations and the nanoparticles induces an immune response mediated by NOX5 activity, against the tumor cells.

In specific embodiments, the method further comprises a step of administering a immunotherapeutic agent prior to, or concomitantly, or after the exposure step to ionizing radiations, in order to further enhance an immune response against the tumor cells in addition or synergy to the immune response induced by the combined effect of ionizing radiations and nanoparticles. Typically, said immunotherapeutic agent may be selected among the immune checkpoint inhibitors, such as PD1/PDL1 inhibitors, CTLA4 inhibitors.

In a specific embodiment, the subject to be treated is selected among the subjects having a tumor resistant to a chemotherapeutic treatment inducing apoptosis.

In specific embodiments, the method further comprises a step of administering a senescence inducer agent in tumor cells, which further enhances senescence in tumor cells, in addition or synergy to the senescence induced by the combined effect of the ionizing radiations and the nanoparticles. Typically, a sublethal dose of a chemotherapeutic agent may be administered as a senescence inducer agent.

In the above method of treatment, non-irradiated cells may be further killed by cellular cannibalism of neighboring irradiated cells.

In specific embodiments, senescence is enhanced by a factor of at least 10%, 20%, 30%, 40% or at least 50%, as compared to senescence induced by the same exposure to ionizing radiations but without the presence of nanoparticles.

Similarly, in specific embodiments, cellular cannibalism is enhanced by a factor of at least 10%, 20%, 30%, 40% or at least 50%, as compared to cellular cannibalism induced by the same exposure to ionizing radiations but without the presence of nanoparticles.

In specific embodiment, the volume of the tumors exposed to the ionizing radiations is smaller than the total volume of the tumor to be treated, for example at least 10% smaller (in volume), or at least 20% smaller (in volume), or at least 30% smaller (in volume), or at least 40% smaller (in volume), or at least 50% smaller (in volume).

The method may further enable the treatment of tumors located outside of the region exposed to the ionizing radiations.

In preferred embodiments, said ionizing radiations are X-ray or γ-ray radiations.

In preferred embodiments, said nanoparticle comprises a rare earth metal, and preferably gadolinium, as a high-Z element. For example, at the time of irradiation, the high-Z element (e.g. gadolinium) concentration in the tumor, may be between 0,1 and 10 µg high-Z element.g⁻¹.

In further preferred embodiments, said nanoparticle comprises chelates of high-Z element, for example rare earth elements.

Typically, said nanoparticle may comprise
- polyorganosiloxane,
- chelates covalently bound to said polyorganosiloxane,
- high-Z elements, for example, rare earth elements, complexed by the chelates.

In the previous embodiment, said chelates may be advantageously selected from the group consisting of: DOTA, DTPA, DTPABA, DOTAGA. For example, said chelates of rare earth elements are chelates of gadolinium, preferably, DOTAGA chelating Gd³⁺. More specifically, the ratio of high-Z elements (for example rare earth elements) per nanoparticle, for example the ratio of gadolinium per nanoparticle, may be between 3 and 100, preferably between 5 and 20.

In specific embodiments, the nanoparticle may be administered intravenously. For example, a single dose between 15 mg/kg and 100 mg/kg of nanoparticles may be injected intravenously in a subject.

In specific embodiments, the nanoparticle is present in the irradiated region of the tumor at a concentration comprised between 0,1 mg/l and 1 g/l, preferably between 0,1 and 100 mg/l. Another aspect of the invention relates to a method of inducing senescence and/or cellular cannibalism of tumor cells and/or an immune response against said tumor cells in a subject in need thereof, said method comprising
a. administering an efficient amount of a suspension of nanoparticles to the tumor of a subject in need thereof, said nanoparticles comprising an element with an atomic Z number higher than 50, and having a mean diameter below 10 nm, preferably below 5 nm, and,
b. exposing said tumor comprising the nanoparticles to an efficient dose of ionizing radiations,
c. wherein the combined effect of the ionizing radiations and the nanoparticles induce senescence and/or cellular cannibalism to the irradiated tumor cells and/or induce an immune response against said tumor cells.

Another aspect of the invention relates to a suspension of nanoparticles for use in the above defined methods of treatment. In particular, the invention relates to a suspension of nanoparticles for use in a method of treating a tumor in a subject in need thereof, the method comprising
a. administering an efficient amount of a suspension of nanoparticles to the tumor of a subject in need thereof, said nanoparticles comprising an element with an atomic Z number higher than 40, preferably higher than 50, and having a mean hydrodynamic diameter below 10nm, preferably below 5 nm, for example between 1 and 5 nm, and,
b. exposing said tumor comprising the nanoparticles to an efficient dose of ionizing radiations,
wherein the combined effect of the ionizing radiations and the nanoparticles induce senescence and/or cellular cannibalism to the irradiated tumor cells.

### Legends to Figures

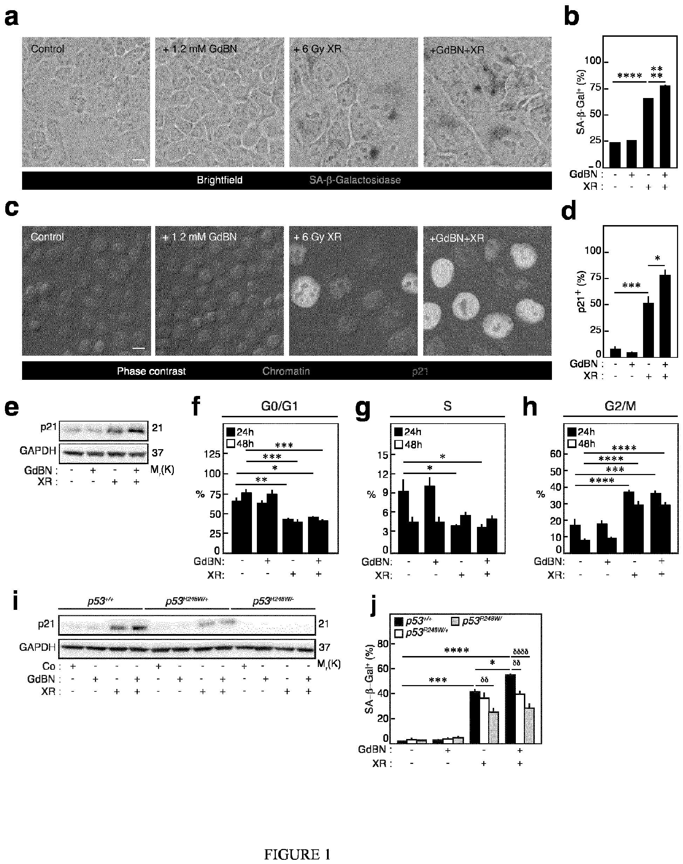

**Figure 1** Gadolinium based nanoparticles (GdBN) sensitize cancer cells to ionizing radiation-elicited senescence. (a,b) Micrographs and frequencies of SA-β-Gal activity observed after the irradiation of HCT116 cells with 6 Gray X-rays (XR) in presence (or in absence) of 1.2 mM gadolinium-based nanoparticles (GdBN+XR) are shown after 48 hours of treatment (scale bar = 10 µm) (n=3). (c,d) Fluorescence micrographs and frequencies of p21 are also shown (scale bar = 10 µm) (n=3). (e) Immunoblot detection of p21 expression after 24-hour treatment is shown. GAPDH is used as loading control. Immunoblots are representative of 3 independent experiments. (f-h) Cell cycle distribution of HCT116 cells that have been irradiated with 6 grays of X-ραψσ (XR) in presence (or in absence) of 1.2 mM GdBN has been analyzed after 24 or 48 hours of culture. Quantitative data of cell cycle analysis are shown (means ± SEM, n = 3). (i) Immunoblot detection of p21 expression on *p53*^{+/+}*, p53*^{*R248W*/+} and *p53*^{*R248W*/*-*} HCT116 cells that have been irradiated with 6 Gray X-rays (XR) in presence (or in absence) of 1.2mM gadolinium-based nanoparticles and analyzed after 24 hours. GAPDH is used as loading control. Immunoblots are representative of 3 independent experiments. (j) Frequencies of SA-β-Gal activity detected after the irradiation of *p53*^{+/+}*, p53*^{*R248W*/+} and *p53*^{*R248W*/-} HCT116 cells with 6 Gray X-rays (XR) in presence (or in absence) of 1.2 mM gadolinium-based nanoparticles (GdBN+XR). The measurement of SA-β-Gal activity was performed 48 hours after the irradiation (means ± SEM, n = 3). Statistical significances are shown. * represents p<0.05, * p<0.01, *** p<0.001 and **** p<0.0001.

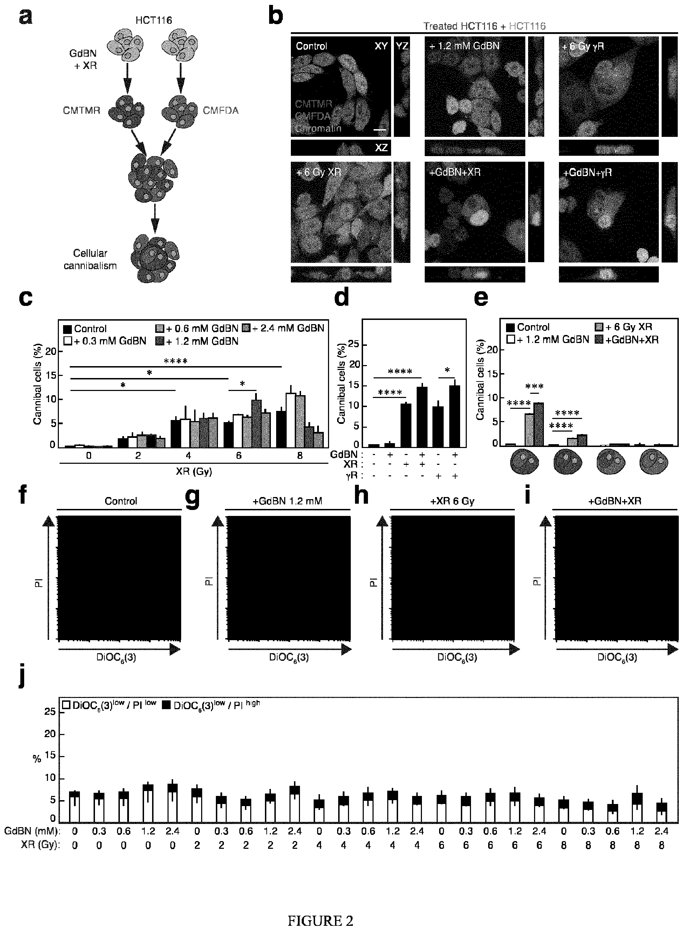

**Figure 2** Detection of GdBN+XR-elicited non-cell autonomous death modalities by confocal fluorescence microscopy. (a) Experimental procedure used to detect cellular cannibalism after the treatment of human colon carcinoma HCT116 cells with XR or GdBN+XR. (b,c) Micrographs and frequencies of cannibal cells detected by confocal fluorescent microscopy after 24 hour co-culture of untreated (green) CMFDA-labeled HCT116 cells with (red) CMTMR-labeled HCT116 cells that have been irradiated with different doses of X-ραψσ in presence (or in absence) of indicated concentrations of gadolinium-based nanoparticles (GdBN) are shown. Representative confocal images of cannibal cells are shown in (b) (scale bar = 10 µm). Frequencies of cannibal cells are in (c) (mean ± SEM, n = 3). (d) Frequencies of cannibal cells detected by confocal fluorescent microscopy after 24 hour co-culture of untreated (green) CMFDA-labeled HCT116 cells with (red) CMTMR-labeled HCT116 cells that have been irradiated with 6 Grays of X-rays (XR) or 6 Grays of γ-rays (γR) in presence (or in absence) of 1.2 mM GdBN are shown. (e) Frequencies of cannibal cells showing R(G), R(R), G(R) or G(G) have been determined and shown (means ± SEM, n = 3). (f-j) Human colon carcinoma HCT116 cells were left untreated (control) (f), treated with 1.2mM GdBN (g), irradiated with 6 Grays of X-rays (XR) (h) of irradiated with irradiated with 6 Grays of X-rays in presence of 1.2mM GdBN (GdBN+XR) and co-stained after 24 hours with DiOC₆(3) and propidium iodide (PI) for the assessment of apoptosis and necrosis-associated parameters. Representative dot plots are shown in (f-i) and quantitative data are shown in (j). (mean ± SEM, n = 3). Statistical significances are shown. * represents p<0.05, *** p<0.001 and **** p<0.0001.

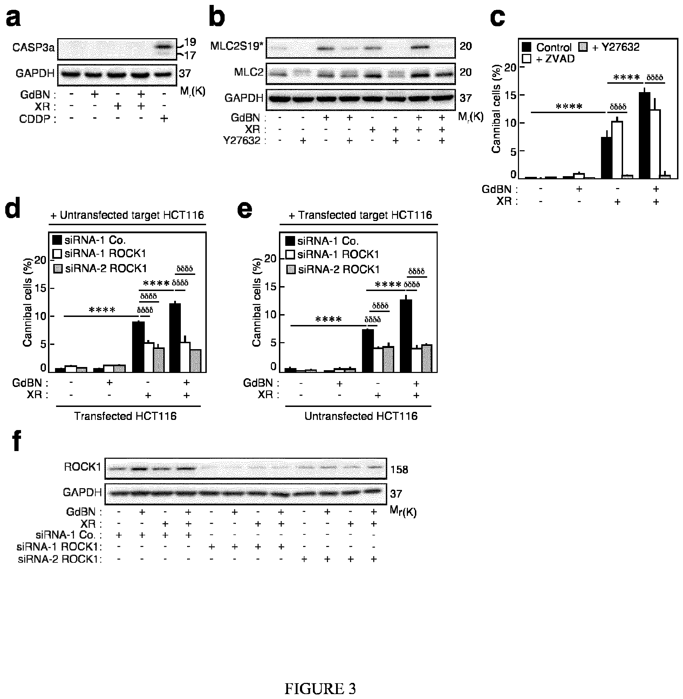

**Figure 3** The activation of ROCK1 is required for XR- and GdBN+XR-mediated cellular cannibalism. (a) Representative immunoblot of the proteolytic cleavage of caspase-3 (CASP3a) detected after the irradiation of human colon carcinoma HCT116 cells with 6 Grays of X-rays (XR) in presence (or in absence) of 1.2mM GdBN is shown. Immunoblots were performed 24 hours after the treatment. GAPDH was used as a loading control (n=3). (b) Representative immunoblots of MLC2 or MLC2S19* of human colon carcinoma HCT116 cells that have been irradiated with 6 Grays of X-rays (XR), in presence (or in absence) of 1.2mM GdBN and cultivated 24 hours after the treatment, with 30 µM of Y27632 during 24 hours are shown. GAPDH was used as a loading control (n=3). (c) The percentage of cannibal cells was determined by confocal microscopy after 24h-homotypic culture of control, XR- or GdBN+XR- treated HCT116 cells in the absence or presence of 30 µM of Y27632 or 100 µM of Z-VAD-fink (n=3). (d) Percentages of cannibal cells detected after 24-hour heterotypic culture of ROCK1 depleted, control, XR- or GdBN+XR-treated HCT116 cells (and control cells) with target HCT116 cells (mean ± SEM, n = 3). (e) Percentages of cannibal cells detected after 24-hour heterotypic culture of control, XR- or GdBN+XR-treated HCT116 cells with target HCT116 cells that have been depleted or not for ROCK1 (mean ± SEM, n = 3). (f) Representative immunoblot of 48-hour ROCK1 depletion in control, XR- and GdBN+XR-treated HCT116 cells is shown. GAPDH was used as a loading control (n=3). Statistical significances are shown. * represents p<0.05, ** p<0.01, *** p<0.001 and **** or δδδδ p<0.0001.

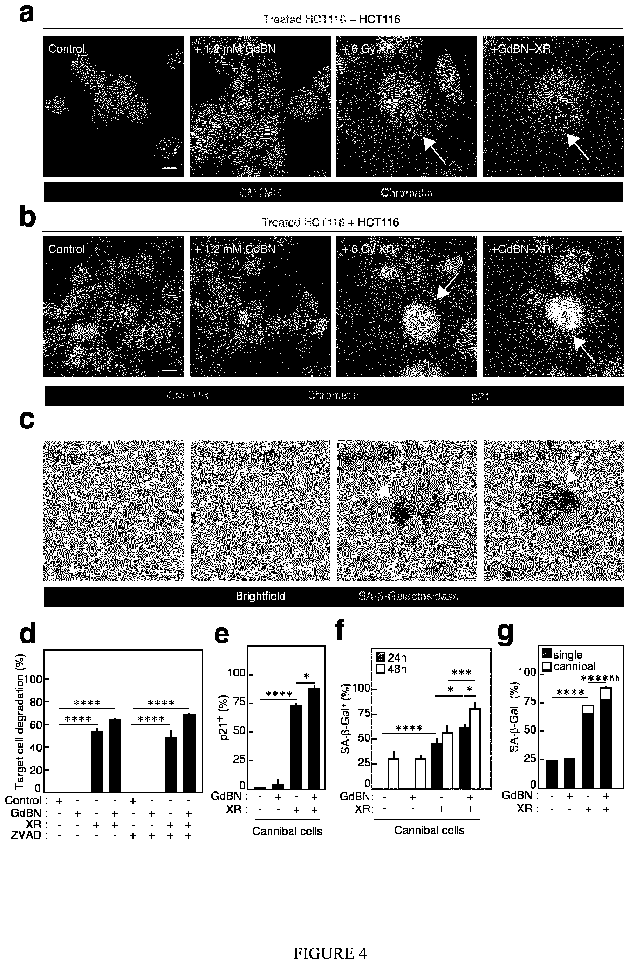

**Figure 4** Detection of the engulfed cell degradation and the senescence in cannibal cells observed after the combined GdBN+XR treatment. (a-c) Representative micrographs and frequencies of target cell degradation (a,d), p21 expression (b,e) and SA-β-Gal⁺ activity (a, c,f and g) detected in single cells and on cannibal cells after 48 hour-homotypic culture of control, XR- or GdBN+XR-treated HCT116 cells in the the presence (or in the absence) of 100 µM of Z-VAD-fink are shown. Before co-culture, treated cells were labeled with (red) CMTMR probe. Arrows indicate target cell degradation (a), p21 expression and SA-β-Gal activity in cannibal cells (c) (scale bar = 10 µm). Frequencies in (d-g) are presented as mean ± SEM (n = 3). Statistical significances are indicated. * represents p<0.05, ** or δδ p<0.01, *** p<0.001 and **** p<0.0001. In Figure 4g, δδ shows statistical significance between XR-treated cannibal cells and GdBN+XR-treated cannibal cells.

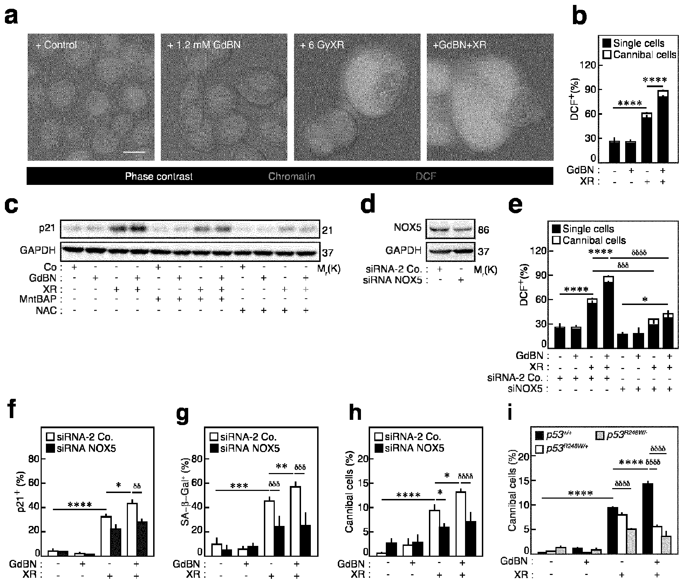

**Figure 5** GdBN+XR-elicited senescence and the cellular cannibalism require a NADPH oxidase 5 (NOX5)-dependent ROS production. (a) Representative micrographs and frequencies of single cells and cannibal cells showing ROS production after the 24 hour co-culture of untreated HCT116 cells with HCT116 cells that have been irradiated with 6 Gy of X-ραψσ in presence (or in absence) of 1.2 mM GdBN are shown. The ROS production is revealed as in (a) by the detection of the conversion of non-fluorescent H2DCFDA probe into green fluorescent DCF⁺ probe using fluorescent microscopy (scale bar = 10 µm). Frequencies of single cells and cannibal cells that exhibited a DCF⁺ staining are in (b) (mean ± SEM, n = 3). (c) Effects of 10 µM of MnTBAP and 100 µM of NAC on p21 expression detected after the treatment of HCT116 cells with 1.2 mM GdBN, 6 Gy XR or GdBN+XR were determined using immunoblots. Representative immunoblots of 3 independent experiments are shown. GAPDH is used as loading control. (d) Validation of NOX5 knockdown after 48-hour transfection of a pool of specific siRNAs. Representative immunoblots of 3 independent experiments are shown. GAPDH is used as loading control. (e-h) Effects of NOX5 depletion on the ROS production (e), the p21 expression (f), the SA-β-Gal activity (g) and cellular cannibalism (h) detected after 24 or 48 hour co-culture of untreated HCT116 cells with HCT116 cells that have been irradiated (or not) with 6 Gy of X-ραψσ in presence (or in absence) of 1.2 mM GdBN were determined by fluorescent microscopy or brightfield microscopy. The frequencies of single cells and/or cannibal cells showing a ROS production (DCF+), a p21 expression, a SA-β-Gal+ activity and cannibalistic activity are shown (mean ± SEM, n = 3). (i) Effects of p53 inactivation on the cellular cannibalism detected after 24 hour co-culture of untreated HCT116 cells with *p53*^{+/+}*, p53*^{*R248W*/+} or *p53*^{*R248W*/*-*} HCT116 cells that have been irradiated (or not) with 6 Gy of X-ραψσ in presence (or in absence) of 1.2 mM GdBN were determined by fluorescent microscopy. The frequencies of single cells and/or cannibal cells are shown (mean ± SEM, n = 3). Statistical significances are indicated. * represents p<0.05, ** p<0.01, *** p<0.001 and **** P<0.0001.

### Detailed Description

The present invention relates to a method of treating a tumor in a subject in need thereof, the method comprising
a. administering an efficient amount of a suspension of nanoparticles to the tumor of a subject in need thereof, said nanoparticles comprising an element with an atomic Z number higher than 40, preferably higher than 50, and having a mean hydrodynamic diameter below 10 nm, preferably below 5 nm, for example between 1 and 5 nm, and,
b. exposing said tumor comprising the nanoparticles to an efficient dose of ionizing radiations,

As used herein, the term "treat" or "treatment" is an approach for obtaining beneficial or desired results, including clinical results. Beneficial or desired results can include but not limited to, alleviation or amelioration of one or more symptoms or conditions, diminishment of extent of disease, stabilized (i.e. not worsening) state of disease, preventing spread of disease, delay or slowing of disease progression, reversal of disease, amelioration or palliation of the disease state, and remission (whether partial or total). In particular, with reference to the treatment of a tumor, the term "treatment" may refer to the inhibition of the growth of the tumor, or the reduction of the size of the tumor.

### The nanoparticles used in the methods according to the invention

The present invention follows from the surprising advantages, demonstrated by the inventors, of a combined effect of certain nanoparticles with ionizing radiations to induce senescence and/or cellular cannibalism and/or immune response against the tumor cells.

The advantageous effects of the method of the present invention are linked in particular to two preferred features of the nanoparticles:
They contain high-Z element to act as a radiosensitizing agent, for example an element with an atomiz Z number higher than 40, for example higher than 50. In specific embodiments, said high-Z element is selected among the heavy metals, and more preferably, Au, Ag, Pt, Pd, Sn, Ta, Zr, Tb, Tm, Ce, Dy, Er, Eu, La, Nd, Pr, Lu, Yb, Bi, Hf, Ho, Pm, Sm, In, and Gd, and mixtures thereof.

They have preferably a very small mean hydrodynamic diameter. Nanoparticles with a mean diameter for example of between 1 and 10 nm, and even more preferably between 1 and 5 nm or for example 1 and 5 nm, typically, around 3 nm, allowing an excellent distribution of these nanoparticles in the tumors, and a rapid renal elimination (and therefore low toxicity) will be advantageously selected.

The size distribution of the nanoparticles is, for example, measured using a commercial particle sizer, such as a Malvern Zêtasizer Nano-S particle sizer based on PCS (Photon Correlation Spectroscopy). This distribution is characterized by a mean hydrodynamic diameter.

For the purposes of the invention, the term "mean hydrodynamic diameter" or "mean diameter" is intended to mean the harmonic mean of the diameters of the particles. A method for measuring this parameter is also described in standard ISO 13321:1996.

In preferred embodiment, the nanoparticles further comprise in addition to the high-Z element, a biocompatible coating. Agent suitable for such biocompatible includes without limitation biocompatible polymers, such as polyethylene glycol, polyethyleneoxide, polyacrylamide, biopolymers, polysaccharides, or polysiloxane.

The nanoparticles can be advantageously used also as an imaging or a contrast agent, for example, in image-guided radiation therapy. The term "contrast agent" is intended to mean any product or composition used in medical imaging for the purpose of artificially increasing the contrast making it possible to visualize a particular anatomical structure (for example certain tissues or organs) or pathological anatomical structures (for example tumors) with respect to neighboring or non-pathological structures. The term "imaging agent" is intended to mean any product or composition used in medical imaging for the purpose of creating a signal making it possible to visualize a particular anatomical structure (for example certain tissues or organs) or pathological anatomical structures (for example tumors) with respect to neighboring or non-pathological structures. The principle of how the contrast or imaging agent operates depends on the imaging technique used.

Advantageously, it will be possible to combine the use of the nanoparticles in the method of treatment of the invention, and for an in vivo detection by MRI, enabling, for example, monitoring of the therapeutic treatment as disclosed in the present invention.

Preferably, only lanthanides, including at least 50% by weight of gadolinium (Gd), of dysprosium (Dy), of lutetium (Lu), for bismuth (Bi) or of holmium (Ho), or mixtures thereof, for example at least 50% by weight of gadolinium, will be chosen as high-Z element in the nanoparticles.

According to one variant, use will be made of nanoparticles in which the part containing lanthanides contains, at its periphery, lanthanides which cause an MRI signal, for example gadolinium, and at least one high-Z element (e.g. Bi) in its central part. Radiation-absorbing high-Z metals with a very high atomic number may therefore be located at the center of the core of the nanoparticle.

In one particular embodiment, the nanoparticles that can be used according to the invention are characterized in that they comprise at least one contrast agent for T1 MRI imaging, and at least one other imaging or contrast agent suitable for one of the following imaging techniques:
- PET or SPECT scintigraphy,
- fluorescence in the visible range or in the near-infrared range,
- X-ray tomodensitometry.

Preferably, the nanoparticles are chosen such that they have a relaxivity r1 per particle of between 50 and 5000 mM⁻¹.s⁻¹ (at 37°C and 1.4 T) and/or a Gd weight ratio of at least 5 %, for example between 5 % and 50 %.

In one specific embodiment, said nanoparticles with a very small hydrodynamic diameter, for example between 1 and 10 nm, preferably between 1 and 5 nm, are nanoparticles comprising chelates of high-Z elements, for example chelates of rare earth elements, and more preferably chelates of gadolinium or bismuth.

In specific embodiments that may be preferably combined with the previous embodiment, said nanoparticles comprises
- polyorganosiloxane,
- chelates covalently bound to said polyorganosiloxane,
- high-Z elements complexed by the chelates.

In a specific embodiment, that may be preferably combined with the previous embodiment, said chelates are selected from the group consisting of DOTA, DTPA, EDTA, EGTA, BAPTA, NOTA, DOTAGA, and DTPABA, and mixtures thereof.

In a specific embodiment, that may be preferably combined with the previous embodiment, said chelates of rare earth element are chelates of gadolinium and/or bismuth, preferably DTPA or DOTAGA chelating Gd³⁺ and/or Bi.

In specific and preferred embodiments, the ratio of high-Z element per nanoparticle, for example the ratio of rare earth elements, e.g. gadolinium (optionally as chelated with DOTAGA) per nanoparticle, is between 3 and 100, preferably between 5 and 20, typically around 10.

For imaging by scintigraphy, the nanoparticles may additionally comprise a radioactive isotope that can be used in scintigraphy, and that is preferably chosen from the group consisting of the radioactive isotopes of In, Tc, Ga, Cu, Zr, Y or Lu, for example: ¹¹¹In, ^{99m}T_{c}, ⁶⁷Ga, ⁶⁸Ga, ⁶⁴Cu, ⁸⁹Zr, ⁹⁰Y or ¹⁷⁷Lu.

For fluorescence in the near-infrared range, the nanoparticles may additionally comprise a lanthanide chosen from Nd, Yb or Er may.

For fluorescence in the visible range, the nanoparticles may additionally comprise a lanthanide chosen from Eu or Tb can be used.

For fluorescence in the near-infrared range, the nanoparticles may additionally comprise an organic fluorophore chosen from Cyanine 5.5, Cyanine 7, Alexa 680, Alexa 700, Alexa 750, Alexa 790, Bodipy.

In specific embodiments, the hybrid nanoparticles are of core-shell type. Nanoparticles of core-shell type, based on a core consisting of a rare earth oxide and of an optionally functionalized polyorganosiloxane matrix are known (see in particular WO 2005/088314, WO 2009/053644).

The nanoparticles may further be functionalized with molecules which allow targeting of the nanoparticles to specific tissues. Said agents can be coupled to the nanoparticle by covalent couplings, or trapped by non-covalent bonding, for example by encapsulation or hydrophilic/hydrophobic interaction or using a chelating agent.

In one specific embodiment, use is made of hybrid nanoparticles comprising:
- a polyorganosiloxane (POS) matrix including, rare earth cations Mⁿ⁺, n being an integer between 2 and 4, optionally partly in the form of a metal oxide and/or oxyhydroxide, optionally associated with doping cations D^{m+}, m being an integer between 2 and 6, D preferably being a rare earth metal other than M, an actinide and/or a transition element;
- a chelate covently bound to the POS via a covalent bond -Si-C-,
- the Mⁿ⁺ cations and, where appropriate, D^{m+} cations being complexed by the chelates;
where appropriate, a targeting molecule, for the targeting of the nanoparticles, said targeting molecule being grafted to the POS or to the chelates.

In the case of a structure of core-shell type, the POS matrix forms the superficial layer surrounding the metal cation-based core. Its thickness can range from 0.5 to 10 nm, and can represent from 25% to 75% of the total volume.

The POS matrix acts as protection for the core with respect to the external medium (in particular protection against hydrolysis) and it optimizes the properties of the contrast agents (luminescence, for example). It also allows the functionalization of the nanoparticle, via the grafting of chelating agents and of targeting molecules.

Advantageously, the chelate is chosen from the following products:
- the products of the group of polyamino polycarboxylic acids and derivatives thereof, and even more preferably in the subgroup comprising: DOTA, DTPA, EDTA, EGTA, BAPTA, NOTA, DOTAGA, DTPABA and mixtures thereof;
- the products of the group comprising porphyrin, chlorine, 1,10-phenanthroline, bipyridine, terpyridine, cyclam, triazacyclononane, derivatives thereof and mixtures thereof; and mixtures thereof.

If M is a lanthanide, e.g. Gd, the chelate is advantageously selected from those which have lanthanide-complexing properties, in particular those of which the complexation constant log(KCl) is greater than 15, preferentially 20. As preferred examples of lanthanide-complexing chelating agents, mention may be made of those comprising a unit of diethylenetriaminepentaacetic acid (DTPA), of 1,4,7,10-tetraazacyclododecane-1,4,7,10-tetraacetic acid (DOTA), or of 1,4,7-triazacyclononane-1,4,7-triacetic acid (NOTA), or derivatives thereof and 1,4,7,10-tetraazacyclododecane-1,glutaric anhydride - 4,7,10-triacetic acid (DOTAGA).

In addition, depending on the intended application, the nanoparticles are optionally doped with another rare earth or actinide metal cation, for example a lanthanide, or even two different lanthanides, at least one being chosen from Eu and Tb.

### "Core-free" ultrafine nanoparticles

In one more particularly preferred embodiment, owing in particular to their very small size and rigid structure, the nanoparticles that can be used according to the invention are obtained by a top-down synthesis route comprising the steps of:
- obtaining a metal (M) oxide core, wherein M is a high-Z element selected from the group of rare earth, an actinide and a transition element,
- adding a polysiloxane shell around the M oxide core, for example via a sol gel process,
- grafting a chelating agent to the POS shell, so that the chelating agent is bound to said POS shell by an -Si-C- covalent bond, thereby obtaining a core-shell precursor nanoparticle, and,
- purifying and transferring the core-shell precursor nanoparticle in an aqueous solution, wherein the grafted agent is in sufficient amount to dissolve the metal (M) oxide core at step d. and to complex the cationic form of (M) thereby reducing the mean hydrodynamic diameter of the resulting hybrid nanoparticle to a mean diameter less than 10 nm, preferably less than 5nm, for example between 1 and 5 nm.

These nanoparticles obtained according to the mode described above do not comprise a core of metal oxide encapsulated by at least one coating. More details regarding the synthesis of these nanoparticles are given in the next section.

This top-down synthesis method results in observed sizes typically of between 1 and 5 nm. The term then used is ultrafine nanoparticles.

These "ultrafine" or "core-free" nanoparticles are optionally grafted to targeting molecules, and in particular molecules targeting lung tissues as described in the following paragraph.

Another characteristic of these ultrafine nanoparticles is the maintaining of the rigid nature of the objects and of the overall geometry of the particles after injection. This strong three-dimensional rigidity is provided by the polysiloxane matrix, where the majority of the silicons are bonded to 3 or 4 other silicon atoms by an oxygen bridge. The combination of this rigidity with their small size makes it possible to increase the relaxivity of these nanoparticles for the intermediate frequencies (20 to 60 MHz) compared with the commercial compounds (Gd-DOTA-based complexes for example), but also for frequencies above 100 MHz present in new-generation high-field MRIs.

Preferably, the nanoparticles for use in the method according to the invention have a relaxivity r1 per Mⁿ⁺ ion greater than 5 mM⁻¹.s⁻¹ (at 37°C) (of Mⁿ⁺ ion), preferentially 10 mM⁻¹.s⁻¹ (at 37°C) (of Mⁿ⁺ ion), for a frequency of 20 MHz. For example, they have a relaxivity r1 per nanoparticle of between 50 and 5000 mM^{-1.}s⁻¹. Even better still, these nanoparticles have a relaxivity r1 per Mⁿ⁺ ion at 60 MHz which is greater than or equal to the relaxivity r1 per Mⁿ⁺ ion at 20 MHz. The relaxivity r1 considered here is a relaxivity per Mⁿ⁺ (for example gadolinium) ion. r1 is extracted from the following formula: 1/T1 = [1/T1]water + r1[Mⁿ⁺]. Further details regarding these ultrafine nanoparticles, the processes for synthesizing them and their uses are described in patent application WO 2011/135101, which is incorporated by way of reference.

### Process for obtaining preferred embodiments of nanoparticles for use according to the invention

Generally, those skilled in the art will be able to easily produce nanoparticles used according to the invention. More specifically, the following elements will be noted:
For nanoparticles of core-shell type, based on a core of lanthanide oxide or oxyhydroxide, use will be made of a production process using an alcohol as solvent, as described for example in P. Perriat et al., J. Coll. Int. Sci, 2004, 273, 191; O. Tillement et al., J. Am. Chem. Soc., 2007, 129, 5076 and P. Perriat et al., J. Phys. Chem. C, 2009, 113, 4038.

For the POS matrix, several techniques can be used, derived from those initiated by Stoeber (Stoeber, W; J. Colloid Interf Sci 1968, 26, 62). Use may also be made of the process used for coating as described in Louis et al. (Louis et al., 2005, Chemistry of Materials, 17, 1673-1682) or international application WO 2005/088314.

In practice, synthesis of ultrafine nanoparticles is for example described in Mignot et al Chem. Eur. J. 2013, 19, 6122-6136: Typically, a precursor nanoparticle of core/shell type is formed with a lanthanide oxide core (via the modified polyol route) and a polysiloxane shell (via sol/gel); this object has, for example, a hydrodynamic diameter of around 10 nm (preferentially 5 nanometers). A lanthanide oxide core of very small size (adjustable less than 10 nm) can thus be produced in an alcohol by means of one of the processes described in the following publications: P. Perriat et al., J. Coll. Int. Sci, 2004, 273, 191; O. Tillement et al., J. Am. Chem. Soc., 2007, 129, 5076 and P. Perriat et al., J. Phys. Chem. C, 2009, 113, 4038.

These cores can be coated with a layer of polysiloxane according to, for example, a protocol described in the following publications: C. Louis et al., Chem. Mat., 2005, 17, 1673 and O. Tillement et al., J. Am. Chem. Soc., 2007, 129, 5076.

Chelating agents specific for the intended metal cations (for example DOTAGA for Gd³⁺) are grafted to the surface of the polysiloxane; it is also possible to insert a part thereof inside the layer, but the control of the formation of the polysiloxane is complex and simple external grafting gives, at these very small sizes, a sufficient proportion of grafting.

The nanoparticles are separated from the synthesis residues by means of a method of dialysis or of tangential filtration, on a membrane comprising pores of appropriate size.

The core is destroyed by dissolution (for example by modifying the pH or by introducing complexing molecules into the solution). This destruction of the core then allows a scattering of the polysiloxane layer (according to a mechanism of slow corrosion or collapse), which makes it possible to finally obtain a polysiloxane object with a complex morphology, the characteristic dimensions of which are of the order of magnitude of the thickness of the polysiloxane layer, i.e. much smaller than the objects produced up until now.

Removing the core thus makes it possible to decrease from a particle size of approximately 5 nanometers in diameter to a size of approximately 3 nanometers. Furthermore, this operation makes it possible to increase the number of M (e.g. gadolinium) per nm3 in comparison with a theoretical polysiloxane nanoparticle of the same size but comprising M (e.g. gadolinium) only at the surface. The number of M for a nanoparticle size can be evaluated by virtue of the M/Si atomic ratio measured by EDX.

Targeting molecules can be grafted onto these nanoparticles for example using coupling by peptide bonding on an organic constituent of the nanoparticle, as described in Montalbetti, C.A.G.N, Falque B. Tetrahedron 2005, 61, 10827-10852.

Use may also be made of a coupling method using "click chemistry", Jewett, J.C.; Bertozzi, C.R. Chem. Soc. Rev. 2010, 39, 1272-1279, and involving groups of the type:
-N3, -CN or -C≡CH, or one of the following groups:

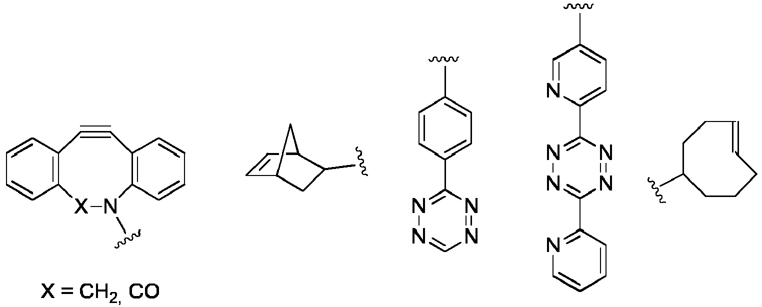

In one specific embodiment, the nanoparticle according to the invention comprises a chelating agent which has an acid function, for example DOTA or DOTAGA. The acid function of the nanoparticle is activated for example using EDC/NHS (1-ethyl-3-(3-dimethylaminopropyl)carbodiimide / N-hydrosuccinimide) in the presence of an appropriate amount of targeting molecules. The nanoparticles thus grafted are then purified, for example by tangential filtration.

### The subject to be treated

The methods according to the present invention are intended to treat tumor of patients, for example tumor of human patient.

The term "patient" and "subject" which are used herein interchangeably refer to any member of the animal kingdom, preferably a mammal, or a human being, including for example a subject that has a tumor.

In specific embodiments, the method of treatment is directed to the treatment of malignant solid tumors, in particular of brain tumors (primary and secondary, glioblastoma..), pelvic malignancies (cervix, prostate, ano rectal and colorectal cancer), liver cancer (primary and secondary), head and neck cancers, lung cancer, eosophagus cancer, breast cancer, pancreatic cancer.

The inventors have identified that the advantageous induction of cellular cannibalism and/or senescence by the combined effect of the nanoparticles and ionizing radiations is mediated by NOX5 and/or ROCK1 activity. Indeed, when inhibiting NOX5 and/or ROCK1 activity *in vitro* in tested cancer cell lines, the induction of cellular cannibalism and/or senescence is not observed.

The patient in need of such treatment may thus be advantageously selected among the patients with tumors with high expression level of NOX5 and/or ROCK1 activity. Those patients are predicted to be good responders to the treatment combining the nanoparticles and ionizing radiations, for inducing senescence and/or cellular cannibalism against said tumor cells to be treated.

As used herein, the term "NOX5" refers to the NADPH oxidase 5 and preferably human NOX5, which generates superoxide. Nox5 interacts with c-abl and superoxide production leads to phosphorylation of c-abl, while inhibition of c-abl kinase activity inhibits Nox5 superoxide production. NOX5 has the protein sequence as identified by reference Q96PH1 in UniProtKB.

As used herein, the term "ROCK1" refers to the Rho-associated coil-coil containing protein kinase 1, which is protein serine/threonine kinase that is activated when bound to the GTP-bound form of Rho. ROCK1 has the protein sequence as identified by reference Q13464 in UniProtKB.

When referring to "NOX5 or ROCK1 expression level or activity", it is referred herein either to, the expression level of the gene (such as mRNA expression) and/or to the corresponding protein expression and/or to the corresponding protein activity (enzymatic activity).

Accordingly, in one preferred embodiment, the method of treatment includes a step of determining NOX5 and/or ROCK1 expression level or activity. A patient is predicted to be a good responder to the treatment of the present invention for example when NOX5 and/or ROCK1 expression level or activity in the tumor of the patient is substantially identical to or higher than a control value.

As used herein, a higher expression level or activity means a statistically significant increase of such expression level or activity as compared to a control value, preferably, at least 10 %, at least 20 %, at least 30 %, at least 40 %, at least 50 % increase of the control value.

The term "good responder" as used herein means that the patient is likely to benefit of a better response to the treatment as compared to a patient with expression level or activity of NOX5 and/or ROCK1 corresponding, for example, to a control value.

The methods of the invention thus comprises the step of (a) determining the expression of NOX5 and/or ROCK1 as predictive biomarkers, in a biopsy or tumor cells obtained from the tumor of said patient and (b) comparing the obtained expression values to corresponding control values.

Said control value may be for example, the mean value of normalized (relative) mean value of NOX5 and/or ROCK1 expression in corresponding tumors of responder patients and/or low-responder patients.

Said control value can also be determined by routine experimentation depending on the quantification methods and the predictive biomarkers that will be used for the methods of the invention.

For example, said control value corresponds to the expression level value observed for low-responder patients, and a patient is predicted to be a responder when the expression level value is statistically higher than the control value.

Alternatively, said control value corresponds to the expression level value observed for responder patients, and a patient is predicted to be a responder when the expression level value is statistically not different or even higher from the control value (threshold value).

Further alternatively, said control value corresponds to the mean value of normalized (relative) mean value of NOX5 and/or ROCK1 expression observed in a non-tumoral tissue of the patient.

The comparison step may be carried out manually or computer assisted.

Expression of the predictive biomarkers NOX5 and/or ROCK1 can be quantified by determining gene or protein expression of such predictive biomarkers in the biological sample of the tumor of a subject. The quantification may be relative (by comparing the amount of a biomarker to a control with known amount of biomarker for example and detecting "higher" or "lower" amount compared to that control) or more precise, at least to determine the specific amount relative to a known control amount.

The terms "nucleic acid" and "polynucleotide" are used interchangeably and refer to a polymeric form of nucleotides of any length, either deoxyribonucleotides or ribonucleotides or analogs thereof. Polynucleotides can have any three-dimensional structure and may perform any function. The following are non-limiting examples of polynucleotides: a gene or gene fragment, exons, messenger RNA (mRNA), cDNA, isolated DNA of any sequence, isolated RNA of any sequence, nucleic acid probes, and primers. A polynucleotide can comprise modified nucleotides, such as methylated nucleotides and nucleotide analogs. If present, modifications to the nucleotide structure can be imparted before or after assembly of the polymer. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any embodiment of this invention that is a polynucleotide encompasses both the double-stranded form and each of two complementary single-stranded forms known or predicted to make up the double-stranded form.

A "gene" refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art. "Gene expression", "gene product" or "expression" are all used herein interchangeably and refer to the nucleic acids or amino acids (e.g., peptide or polypeptide) generated when a gene is transcribed and translated, cDNA or RNA sequence of the biomarker; biomarker gene expression, biomarker protein expression, biomarker mRNA expression; functional effect of the biomarker protein, functional effect of the biomarker gene, cDNA or mRNA, protein, cDNA, gene or mRNA activity.

In a particular embodiment "expression level" "gene expression", "gene product" or "expression" denotes mRNA expression, cDNA expression, protein transcription and protein expression.

The term "polypeptide" is used interchangeably with the term "protein" and in its broadest sense refers to a compound of two or more subunit amino acids. The subunits can be linked by peptide bonds.

Such quantification methods may alternatively include detection and quantification of the corresponding gene expression level of said predictive biomarker which encompasses the quantification of corresponding mRNA of said predictive biomarker, for example by performing Real-Time quantitative PCR, as well as by using DNA microarrays, i.e. substrate onto which are bound nucleic acids, at defined position, that specifically hybridize with the cDNA corresponding to amplified mRNA of said predictive biomarker.

Typically, in specific embodiments, a mixture of transcribed polynucleotides (mRNA) obtained from the biological sample of the patient is subjected to reverse transcription and quantitative amplification. Said cDNA or mRNA may be detected by *in vitro* techniques either by stringent hybridization to DNA microarrays or Northern blots.

In any cases, a general principle of such detection and quantification assays involve preparing a sample or reaction mixture that may contain a predictive biomarker and a probe under appropriate conditions and for a time sufficient to allow the predictive biomarker and probe to interact and bind, thus forming a complex that can be detected (and quantified) in the reaction mixture.

These detection and/or quantification assays of a biomarker can be conducted in a variety of ways. Appropriate conditions to the particular assay and components thereof will be well known to one skilled in the art.

In a particular embodiment, the level of predictive biomarker mRNA can be determined both by in vitro formats in a biological sample using methods known in the art.

Specific methods include without limitations PCR, RT-PCR, RT-qPCR or Northern blot.

Expression level of the biomarker can also be determined by examining protein expression or the protein product of at least one of the predictive biomarkers. Determining the protein level involves measuring the amount of any immunospecific binding that occurs between an antibody that selectively recognizes and binds to the polypeptide of the biomarker in a sample obtained from a patient and comparing this to the amount of immunospecific binding of at least one biomarker in a control sample. The amount of protein expression of the biomarker can be increased or reduced when compared with control expression.

Various methods are known in the art for detecting protein expression levels in such biological samples, including various immunoassays methods. They include but are not limited to radioimmunoassays, ELISA (enzyme linked immunosorbent assays), "sandwich" immunoassays, immunoradiometric assays, in situ immunoassays (using e.g., colloidal gold, enzyme or radioisotope labels), western blot analysis, immunoprecipitation assays, immunofluorescent assays, flow cytometry, immunohistochemistry, confocal microscopy, enzymatic assays, surface plasmon resonance and PAGE-SDS. NOX5 or ROCK1 elisa kits are commercially available.

Alternatively, the enzymatic activity of NOX1 and/or ROCK1 activity may be measured as a predictive biomarker, using appropriate enzymatic assays. A rock1 kinase assay is available for example from PROMEGA (ADP-Glo^{™} Kinase Assay).

### Administering the nanoparticles to the subject to be treated

The method of the present invention comprises a step of administering an efficient amount of a suspension of the nanoparticles to the tumor of the subject.

The nanoparticles can be administered to the subject using different possible routes such as local (intra-tumoral (IT), intra-arterial (IA)), subcutaneous, intravenous (IV), intradermic, airways (inhalation), intra-peritoneal, intramuscular, intra-thecal, intraocular or oral route.

In specific embodiments, the nanoparticles are administered intravenously, and the nanoparticles are advantageously targeted to the tumors, by passive targeting, for example by enhanced permeability and retention effect.

Repeated injections or administrations of nanoparticles can be performed, when appropriate. In one embodiment, the nanoparticles is administered to the patient an amount so that, at the time of irradiation of the tumor, the high-Z element (e.g. gadolinium) concentration in the tumor, is between 0,1 and 10µg high-Z element.g⁻¹.

In a specific embodiment, a single dose between 15 mg/kg and 100 mg/kg of nanoparticles (for example the coreless ultrafine nanoparticles with chelates of gadolinium) is injected intravenously in a subject.

In a specific embodiment, the nanoparticles is administered to the tumor of the patient so that, the nanoparticle is present in the irradiated region of the tumor at a concentration between 0,1mg/l and 1g/l, preferably between 0,1 and 100mg/l.

Since the effect of induction of senescence and/or cellular cannibalism is mediated by NOX5 and/or ROCK1 activity, it may be advantageous to further administer an enhancer agent of NOX5 and/or ROCK1 activity to the patient.

Accordingly, in a specific embodiment of the method of treatment, the method of treatment further includes a step of administering an enhancer agent of NOX5 and/or ROCK1 activity, prior to, or concomitantly, or after the exposure step to ionizing radiations.

As used herein, an enhancer agent of NOX5 and/or ROCK1 activity, refers to a compound or drug or combination of compounds or drugs that can increase NOX5 and/or ROCK1 activity in vivo, for example, in the tumor tissue of the patient. Such enhancer agent may be a direct activator of the oxidase activity of NOX5 or kinase activity of ROCK1 or an indirect enhancer, acting for example downstream of the signaling pathways of NOX5 or ROCK1 respectively.

Examples of such enhancer agents of NOX5 include ciplatin, calcium influx, phorbol myristate acetate, Angiotensin II and endothelin-1.

The inventors have further found that the combined effect or ionizing radiations and the nanoparticles may induce an immune response mediated by NOX5 activity, against the tumor cells. Such response may be directed to the cells of the irradiated tumors, or to other tumor cells within the patient.

It may be therefore advantageous to increase such immune response for example by combining the method of treatment with an immunotherapeutic treatment. Accordingly, in a specific embodiment, the method of the present invention further comprises a step of administering an immunotherapeutic agent prior to, or concomitantly, or after the exposure step to ionizing radiations, to further enhance the immune response in addition or synergy to the immune response induced by the combined effect of ionizing radiations and nanoparticles.

Typically, said immunotherapeutic drug is selected among the immune checkpoint inhibitors. Immune checkpoint inhibitors are described for example in Parldoll DM Nat Rev Cancer 12(2012):252-264 and Herrera FG et al CA Cancer J Clin. 67(2017):65-85. doi: 10.3322/caac.21358. These include for example PD1/PDL1 inhibitors, CTLA4 inhibitors, and more specifically, anti-PD1 or anti-PDL1 antibodies and/or anti-CTLA4 antibody.

One of the major finding of the inventors is that the combined effect of nanoparticles and ionizing radiations induce cell death of tumors by a mechanism that is not related to apoptosis (non-cell autonomous cell death mechanism). Therefore, the method of treatment of the present invention may be more particularly suitable for treating tumors that have been shown to be resistant to usual chemotherapeutic treatments inducing apoptosis of tumor cells. Such usual chemotherapeutic treatment includes in particular cisplatinum and derivatives, 5 Fluor-uracile, taxanes, and EGFr inhibitors.

The method of treatment of the invention may further comprise a step of administering a senescence inducer agent. Such senescence inducer agent may advantageously enhance senescence in addition or synergy to the senescence induced by the combined effect of ionizing radiations and the nanoparticles. In specific embodiment, said senescence inducer agent is selected among the chemotherapeutic agents, known to induce senescence, including without limitation:
Actinomycin, all-trans retinoic, acid Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, HydroxyureaI, darubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine.

### Exposing the tumor to ionizing radiations

The nanoparticles herein described will be used to treat tumors where radiotherapy is a classical treatment or is the most appropriate treatment or could be indicated.

Radiation therapy or radiotherapy is the medical use of irradiation -i.e. ionizing radiation- as part of cancer treatment to control malignant cells. It is used as palliative treatment or as therapeutic treatment. Radiotherapy is accepted as an important standard therapy for treating various types of cancers.

As used herein, the term "radiotherapy" is used for the treatment of diseases of oncological nature with irradiation corresponding to ionizing radiation. Ionizing radiation deposits energy that injures or destroys cells in the area being treated (the target tissue) by damaging their genetic material, making it impossible for these cells to continue to grow.

In specific embodiment, the method of the invention comprises exposing the tumor comprising the nanoparticles to an efficient dose of ionizing radiations, wherein said ionizing radiations are photons, e.g. X-rays. Depending on the amount of energy they possess, the rays can be used to destroy cancer cells on the surface of or deeper in the body. The higher the energy of the X-ray beam, the deeper the X-rays can go into the target tissue. Linear accelerators and betatrons produce X-rays of increasingly greater energy. The use of machines to focus radiation (such as X-rays) on a cancer site is called external beam radiotherapy.

In an alternative embodiment of the method of treatment according to the invention, gamma rays are used. Gamma rays are produced spontaneously as certain elements (such as radium, uranium, and cobalt 60) release radiation as they decompose, or decay.

Ionizing radiations are typically of 2keV to 25000 keV, in particular of 2 keV to 6000 keV (i.e. 6 MeV) or of 2 keV to 1500 keV (such as cobalt 60 source).

A person of ordinary skill in the radiotherapy art knows how to determine an appropriate dosing and application schedule, depending on the nature of the disease and the constitution of the patient. In particular, the person knows how to assess dose-limiting toxicity (DLT) and how to determine the maximum tolerated dose (MTD) accordingly.

The amount of radiation used in photon radiation therapy is measured in gray (Gy), and varies depending on the type and stage of cancer being treated. For curative cases, the typical dose for a solid epithelial tumor ranges from 60 to 80 Gy. Many other factors are considered by radiation oncologists when selecting a dose, including whether the patient is receiving chemotherapy, patient co-morbidities, whether radiation therapy is being administered before or after surgery, and the degree of success of surgery.

The total dose is typically fractionated (spread out over time). Amount and schedules (planning and delivery of ionizing radiations, fraction dose, fraction delivery schema, total dose alone or in combination with other anti-cancer agents etc) is defined for any disease/anatomical site/disease stage patient setting/age and constitutes the standard of care for any specific situation.

A typical conventional fractionation schedule for adults may be 1.8 to 2 Gy per day, five days a week, for example for 5 to 8 consecutive weeks.

Considering the combined effect of nanoparticles and ionizing radiations according to the present method obtained with high dose of ionizing radiations, in one specific embodiment, the dose of ionizing radiations exposed to the tumor of the patient is advantageously hypo fractionated. For example, a dose per fraction of at least 3 Gy, and for example between 3 Gy and 9 Gy, or between 5 and 7 Gy is exposed to the tumor of the patient and radiation total dose is delivered in few fractions (typically, but not necessarily no more than 10 fractions).

The inventors have established that the combined treatment with the nanoparticles and radiotherapy enables to induce an effect also on tumor cells which have not been irradiated, in particular via induction of cellular senescence, cellular cannibalism and/or immune response on such cells.

According to the advantageous effect, the treatment thus may typically enable enhancement of senescence by a factor of at least 10 %, 20 %, 30 %, 40 % or at least 50 %, as compared to senescence induced by the same exposure to ionizing radiations but without the presence of nanoparticles.

According to the advantageous effect, the treatment thus may also typically enable enhancement of cellular cannibalism by a factor of at least 10 %, 20 %, 30 %, 40 % or at least 50 %, as compared to cellular cannibalism induced by the same exposure to ionizing radiations but without the presence of nanoparticles.

Therefore, in one embodiment, the volume of the tumors exposed to ionizing radiations is smaller than the total volume to be treated, for example at least 10 %, 20 %, 30 %, 40 %, or at least 50 % smaller (in volume).

Additionally, in certain embodiments, the method enables the treatment of tumors located outside of the region exposed to ionizing radiations.

The nanoparticles may be administered e.g. , 5 minutes, 15 minutes, 30 minutes, 45 minutes, 1 hour, 2 hours, 4 hours, 6 hours, 12 hours, 24 hours), prior to the administration of the first irradiation of radiotherapy, to the subject with the tumor to be treated.

Other aspects and advantages of the method of the invention will become apparent in the following examples, which are given for purposes of illustration only.

### Examples

### Materials and Methods

### Cells, reagents and Gadolinium-based nanoparticles

Colorectal carcinoma (p53^{+/+}, p53^{R248W/+} and p53^{R248/-}) HCT116 cells were maintained in McCoy's 5A medium (Life Technology) supplemented with 10% heat-inactivated fetal bovine serum (Hycultec GmbH), 2mM L-glutamine and 100 IU/mL penicillin-streptomycin (Life technology). Human colon carcinoma mutant HCT116 *p53*^{*R248W*/}*⁻, p53*^{*R248W*/+} and HCT116 *p53*^{+/+} were obtained from Dr. Christophe Bourdon. The benzyloxycarboxyl-Val-Ala-Asp (OMe) fluoromethylketone (Z-VAD-fmk) was obtained from Bachem. The ROCK1 inhibitor (Y27632) and the N-acetylcysteine (NAC) were from Sigma and the Manganese (III)-tetrakis(4-benzoic acid) porphyrin (MnTBAP) from Merck chemicals. Gadolinium-based Nanoparticle (GdBN) were obtained from NH TherAguix.

### RNA -mediated interference

The small interfering RNAs (siRNAs) specific for ROCK1 (siRNA-1 ROCK1: 5' GCC GCC GGG ACC CAA CUA U 3'; siRNA-2 ROCK1: 5' GGA AUC CAG UUG AAU ACA A 3') and control siRNA (siRNA-1 Co.:5' GCC GGU AUG CCG GUU AAG U 3') were obtained from Sigma. The SMARTpool siGENOME NOX5 siRNA (siRNA NOX5) (D-010195-05) contains 4 siRNA (siRNA-1 : 5' GGA GCA AGG UGU UCC AGA A 3'; siRNA-2: 5' CUA UAG ACC UGG UGA CUA C 3' ; siRNA-3 : 5' GCU UAU GGG CUA CGU GGU A 3' and siRNA-4: 5' CCU UCU UUG CAG AGC GAU U 3'). The control siGENOME Non-Targeting siRNA (indicated as siRNA-2 Co.) is a pool of four on-target plus non-targeting siRNAs (D-001206-13-05). SMARTpool siGENOME NOX5 siRNA and siGENOME Non-Targeting siRNA Pool #1 were purchased from Dharmacon. For RNA interference, HCT116 cells were seeded (5.0x10⁵ cells/2 mL/well in 6-well plate) 48 hours before siRNAs transfection. Then, cells were transfected with 10 nM siRNAs using Lipofectamine RNAi max (#13778150, Life technologies) according to the manufacturer's instructions and incubated at 37°C for 24h before subsequent experiments.

### Irradiation

HCT116 cells were seeded in 6-well plates and incubated at 37°C during 1 hour with indicated concentrations of GdBN. Then, cells were irradiated with X-ray irradiator (1 Gy/min, 200 keV, 15 mA, 2 mm copper thickness, X-RAD 320, Precision X-Ray) or with γamma-ray irradiator (IBL-637, Cs¹³⁷, 1 Gy/min, gamma CIS-BioInternational, IBA, Saclay, France). Cells were harvested at indicated time points after irradiation for subsequent experiments.

### Immunof luorescence and flow cytometry

Cellular cannibalism was determined as previously described ¹³. Briefly, treated cells were stained with 10 µM of 5-(and-6)-(((4-Chloromethyl)Benzoyl)Amino)Tetramethylrhodamine (Cell Tracker Orange CMTMR, Invitrogen) and untreated cells were stained with 10 µM of 5-chloromethylfluorescein diacetate (Cell tracker Green CMFDA, Invitrogen). Cells are then co-cultured for the indicated time, in presence of the pharmacological inhibitor of ROCK, Y27632 (30µM, TOCRIS) or the pan-caspase inhibitor, zVAD-fmk (100 µM, Calbiochem). For specific subcellular staining of the cyclin-dependent kinase inhibitor p21 on single and cannibal cells, cells were fixed in 4% paraformaldehyde/ phosphate-buffered saline (PBS) for 10 minutes, permeabilized in 0,3% Triton X-100 (Sigma) in PBS and incubated with 5% FCS-PBS for 1 hour. Rabbit antibody against human p21 (form Cell Signaling Technology, #2947) was used for immunodetection in PBS containing 1 mg/ml BSA and revealed with goat anti-rabbit IgG conjugated to Alexa 488 fluorochrome from Invitrogen. Cells were counterstained with Hoechst 33342 (Invitrogen) and analyzed by fluorescent confocal microscopy on a Zeiss LSM510 or by fluorescent microscopy on a LEICA DMi8 using a 63× objective. To detect the induction of cell death by flow cytometry, cells were stained with 3,3'-dihexyloxacarbocyanine, DIOC₆(3) at 40 nM and propidium iodide at 2 µg/mL (Invitrogen, Molecular probes, OR, USA) for 30 min at 37°C and 5% CO₂. Cytofluorometric determinations were carried out on Guava EasyCyte (Millipore EMD) and data were analyzed by means with Incyte software (Millipore). Cell cycle distributions were assessed with 10µg/ml of Hoechst 33342 (Invitrogen), as previously described ¹⁴. Cell fluorescence was quantified using LSR II^{™} flow cytometer (Becton-Dickinson). Fluorescence histograms were analyzed with Kaluza software version 1.5.

### Detection of senescence associated β-galactosidase

At the indicated time, cells were fixed and stained using the senescence β-galactosidase staining kit (Cell signaling technologies) as previously described ¹³.

### Immunoblotting

Total cell lysates were prepared in lysis buffer (0,1% NP40 ; 200 mM HEPES ; 100 mM KCl; 1 mM EDTA; 1% Glycerol final concentration) supplemented with proteases and phosphatases inhibitors cocktail (EDTA-free inhibitors, Roche-diagnostics, Meylan, France). Protein extracts were quantified using the Bradford assay (Bio-Rad Laboratories, Hercules, CA, USA). Protein extracts (20-40 µg) were run onto 4-12% NuPAGE Bis-tris gel (Invitrogen) and transferred onto nitrocellulose membrane at 4°C. After blocking, membranes were incubated overnight at 4°C with primary antibodies according to the manufacturer's instructions: p21^{WAF1/CIP1} (Cell signaling #2947); NOX-5 (Abcam #191010); cleaved caspase-3 (Cell signaling #9664); MLC2S19* (Cell signaling #3671) or MLC2 (Cell signaling #8505). Horseradish peroxidase-conjugated goat anti-mouse or anti-rabbit (Southern Biotechnology) antibodies were then incubated during 1 hour and revealed with the enhanced chemiluminescence prime detection (Amersham, GE-Healthcare) using direct chemiluminescence image scanning (G :BOX Syngene). GAPDH (#MAB374, EMD Millipore) was used as loading control.

### Detection of ROS production.

Cells were seeded on cover glass dishes (World Precision Instruments). Intracellular ROS levels were evaluated using the fluorescent probe (2,7-dichlorohydro-fluorescein diacetate (H₂DCFDA) according to the manufacturer's instructions (Invitrogen). Cells were counterstained with Hoechst 33342 (Invitrogen) and analyzed by fluorescent microscopy (Leica DMi8 using a 63x objective).

### Statistical analysis

No statistical method was used to predetermine the sample size. Statistical significances were determined using the one-way ANOVA test. Statistically significant values are reported in figure legends. All experiments were independently performed at least three times. Data are expressed as mean ± SEM. GraphPad Prism version 6.0b (GraphPad Software) was employed to perform statistical analysis.

### RESULTS

### The combination of gadolinium-based nanoparticles with ionizing radiation induces cellular senescence

As we previously reported,^{13,14} cancer cells are eliminated after ionizing radiation through distinct lethal modalities (including NCAD or CAD deaths) that may be induced simultaneously to cellular senescence. To determine the biological effects of the irradiation of cancer cells in presence of gadolinium-based nanoparticles, we first analyzed the ability of this treatment to induce the senescence of cancer cells. Thus, human colorectal HCT116 cells were pre-treated with 1.2 mM of gadolinium-based nanoparticles (GdBN) during 1 hour and then, irradiated with one single dose of 6 Gy X-rays (XR). After 2 days, the cytoplasmic senescence-associated β-galactosidase (SA-β-Gal) activity, the expression of the cyclin-dependent kinase inhibitor protein p21 and the cell cycle progression of treated HCT116 cells were analyzed. We detected a significant increase of the frequency of SA-β-Gal⁺ cells after the treatment of HCT116 cells with XR, as compare to control, and also paid a particular attention to the fact that the combination of GdBN+XR significantly increases the number of SA-β-Gal⁺ cells detected after treatment with XR (Figures 1a and 1b). These results, which were confirmed by detecting the increased expression of p21 by fluorescence microscopy (Figures 1c and 1d) and by western blot (Figure 1e), demonstrate that the combination of gadolinium-containing particles with ionizing radiation sensitizes cancer cells to the induction of senescence after XR. In parallel, we also analyzed using flow cytometry the cell cycle distribution of HCT116 cells that have been irradiated with 6 Gy alone or in combination with 1.2 mM GdBN and cultivated during 24 and 48 hours. As compared to control HCT116 cells, a significant accumulation in G2/M phase of HCT116 cells that have been treated with XR alone or with GdBN+XR was detected after 24 hours, but not after 48 hours of incubation (Figures 1f-1h). These results are consistent with the significant increase of p21 expression that we detected after the association of XR with GdBN (Figures 1d and 1e). Considering that the transcription factor p53 may cause through the control of p21 expression, a prolonged G2 arrest during the induction of cellular senescence ^{15, 16}, HCT116 cells that are wild-type (*p53*^{+/+}), mutated or/and transcriptionally inactivated for p53 (*p53*^{*R248W*/+} or *p53*^{*R248W*/*-*}) have been irradiated with 6 Gy alone or in combination with 1.2 mM GdBN and analyzed after 48 hours for p21 expression and SA-β-Gal activity. As predicted, we observed that p21 expression (Figure 1f) and SA-β-Gal activity (Figure 1g) were significantly reduced after XR and GdBN+XR treatments, indicating that the transcriptional activity of p53 is required for both XR- or XR+GdBN-induced cellular senescence. Altogether, these results demonstrate that the irradiation of cancer cells in presence of gadolinium-containing nanoparticles favors the elimination of irradiated cancer cells through the induction of cellular senescence.

### The gadolinium-based nanoparticles favors the cellular cannibalism of irradiated cancer cells

To further identify lethal processes that are induced by the combined treatment of cancer cells with GdBN and XR, we determined the ability of this treatment to induce cell-autonomous and non-cell autonomous deaths. In this context, as previously described ^{13, 14}, we first performed co-cultures between untreated HCT116 cells that have been labeled with 5-(and-6)-(((4-chloromethyl)benzoyl)amino) tetramethylrhodamine (CMTMR, red) fluorescent vital probe and isogenic HCT116 cells that have been labeled with 5-chloromethylfluorescein diacetate (CMFDA, green) fluorescent vital probe, and irradiated with different doses in presence (or in absence) of indicated concentrations of GdBN. After 24 hours of co-culture, cell fates of CMFDA⁺ cells and CMTMR⁺ cells were analyzed for cell engulfment. Confocal analysis showed that during co-cultures of irradiated (XR) or GdBN+XR-treated CMTMR⁺ HCT116 cells with untreated CMFDA⁺ HCT116 cells (Figure 2b), cell engulfment was detected. This process occurred in dose dependent manner (Figure 2c) and is also observed when cancer cells were irradiated with γ-irradiation (Figure 2d). To further characterize the biological mechanisms involved in the cellular engulfment, we examined the ability of these treatments to induce either cellular invasion or cellular cannibalism mechanisms. Using fluorescence microscopy, we observed that irradiated (XR) and GdBN+XR-treated CMTMR⁺ HCT116 cells internalized neighboring (CMFDA⁺ or CMTMR⁺) HCT116 cells independently of the treatment that they received (Figure 2e). We noticed a significant increase of the cannibalistic activity of GdBN+XR-treated cells as compared to irradiated cells, revealing the combined treatment of cancer cells with GdBN and XR increases the ability of irradiated cells to exhibit cannibalistic activity. In parallel to these experiments, we also evaluated the ability of irradiated cells to simultaneously undergo other CAD modalities (such as apoptosis and necrosis). By analyzing the permeabilization of mitochondrial membranes and plasma membranes of treated cells (through the simultaneous detection of *3,3'-*dihexyloxacarbocyanine iodide and propidium iodide (DiOC₆(3)/IP) stainings by flow cytometry), we observed that the cellular cannibalism that we detected in response to XR or GdBN+XR treatment was induced in absence of significant induction of apoptosis or necrosis (Figure 2f-2j). Altogether, these results underline the fact that the cellular cannibalism of irradiated cells can be enhanced after the treatment of cancer cells with gadolinium-containing nanoparticles.

### The activation of ROCK1 kinase is required for the live cell engulfment detected after GdBN+XR treatment

To precise the molecular mechanisms involved in the cellular cannibalism that we described above, we then studied the signaling pathways that are associated with the live cell engulfment of neighboring cancer cells. We first revealed that the cellular engulfment that was observed after XR or GdBN+XR treatment, occurred in absence of cell death induction (as revealed by the absence of pro-apoptotic cleavage of caspase-3 after XR or GdBN+XR treatment (Figure 3a)) and was not inhibited by pan-caspase inhibitor, Z-VAD-fink (Figure 3c), indicating that this process is independent of the apoptotic uptake of dying cells and allows the internalization of live cancer cells. In addition, we showed that the treatment of HCT116 cells with GdBN, XR, or the combined treatment with GdBN and XR activated the kinase ROCK1 (as revealed by the phosphorylation of myosin light chain 2 on serine 19 (MLC2S19*)) (Figure 3b). We observed the significant increased phosphorylation of MLC2S19* after the irradiation of cancer cells in presence or in absence of GdBN (Figure 3c), indicating that the activation of ROCK1 may be required for the engulfment of neighboring cancer cells detected after GdBN+XR treatment. To determine the contribution of the ROCK-1 dependent signaling pathway during XR- or GdBN+XR-mediated cellular engulfment, we then added the pharmacological inhibitor of ROCK1 (Y27632) to the co-cultures of HCT116 cells that have been irradiated with 6 Gy in presence (or in absence) of 1.2 mM GdBN and determined the frequency of cellular cannibalism after 24 hours of co-culture. We observed that the pharmacological inhibition of ROCK1 by Y27632 (as revealed in Figure 3b) inhibited the cellular cannibalism detected after 6 Gy irradiation alone and after the irradiation of HCT116 cells in presence of 1.2 mM GdBN (Figure 3c), revealing that the biological activity of ROCK1 is required for the cellular cannibalism that is detected after XR or GdBN+XR treatment. We then better defined the contribution of the kinase ROCK1 on GdBN+XR-elicited cellular cannibalism by depleting ROCK1 with two specific small interfering RNA (siRNA) on either engulfed (target) cells or on engulfing (cannibal) cells. We observed that the depletion of ROCK1 on both interacting cells reduced the frequency of cellular cannibalism detected after XR or GdBN+XR treatment (Figures 3d and 3e), indicating that the kinase ROCK1 plays a central role in both engulfed target cells and engulfing cannibal cells during the induction of GdBN+XR-mediated cellular cannibalism.

### The degradation of engulfed cells positively correlates with the senescence of cannibal cells

To determine the fate of both engulfed cells and cannibal cells detected after XR or GdBN+XR treatment, we detected through fluorescence microscopy, the presence of chromatin condensation and nuclear fragmentation in the nuclei of engulfed HCT116 cells observed after 24 hour co-culture of HCT116 cells that were irradiated with 6 Gy in presence (or in absence) of 1.2 mM GdBN was determined. We observed that all engulfed cells detected after the irradiation alone or after the irradiation of cancer cells in presence of GdBN, exhibited signs of target cell degradation (Figures 4a and 4d). Interestingly, we demonstrated that the pan-caspase inhibitor inhibitor, Z-VAD-fink failed to repress target cell degradation (Figure 4d), indicating that the activation of caspases is not required for the execution of the cellular cannibalism elicited by the X-rays radiation or its combination with GdBN. In addition, we also revealed that XR- or GdBN+XR-elicited cannibal cells exhibited an increase of p21 expression (Figures 4b and 4e) and SA-β-Gal⁺ activity (Figures 4c and 4f), indicating that after XR- or GdBN+XR treatment, cannibal cells may undergo entescence or alternatively, that single senescent cells may internalize neighboring cells. To determine whether the induction of entescence or the activation of cannibalistic activity of senescent cells is associated with the cannibalistic activity detected after XR or GdBN+XR treatment, we determined the frequency of single cells and cannibal cells that showed SA-β-Gal⁺ activity after XR or GdBN+XR treatment. We observed that the vast majority of SA-β-Gal⁺ cells are single cells (Figure 4g), suggesting that cellular cannibalism seems to occur after the induction of cell-autonomous senescence of cancer cells that have been treated with XR or GdBN+XR.

### GdBN+XR mediated senescence and cellular cannibalism are controlled by a NADPH oxidase 5 (NOX5)-dependent ROS production

Considering that the reactive oxygen species (ROS) production is one of the major cellular stresses involved in the induction of senescence after ionizing radiation ¹⁷, we analyzed the ROS production after XR and GdBN+XR treatments. By detecting with fluorescence microscopy the conversion of the non-fluorescent dye 2,7-dichlorohydro fluorescein diacetate (H₂DCFDA) into fluorescent 2,7-dichlorohydro fluorescein (DCF), we determined the ability of single cells and cannibal cells obtained after XR or GdBN+XR treatment to generate ROS. We observed that these treatments induced the production of ROS in both single and cannibal cells (Figures 5a and 5b). Moreover, we revealed that the antioxidant N-acetylcysteine (NAC) and the superoxide dismutase (SOD) mimetic Mn(III)tetrakis (4-benzoic acid) (MnTBAP), which are known to blunt the ROS production ¹⁸, reduced the cellular senescence (as revealed by the detection of p21 expression (Figure 5c)) that is detected after the treatment of HCT116 cells with XR alone or with the ΓδBN+XR combination. These results demonstrated that the ROS production plays a key role for the induction of the senescence and the cellular cannibalism associated with XR and GdBN+XR treatments.

To determine whether the NADPH oxidases (NOX), which are the major intracellular sources of ROS production, may regulate these processes ^{19, 20}, we determined whether the NADPH oxidase 5 (NOX5) - a NADPH oxidase that has been involved in the death of irradiated human primary fibroblasts ²¹ - may participate to the production of ROS. Thus, we evaluated the effects of NOX5 depletion on the ROS production, the p21 expression, the SA-β-Galactosidase activity and the cellular cannibalism that are detected after the treatment of HCT116 cells with XR or ΓδBN+ΞP (Figures 5a-5h). We demonstrated that the depletion of NOX5 with specific small interfering RNA blunted the ROS production (Figures 5d and 5e), reduced the p21 expression (Figure 5f) and the SA-β-Gal⁺ activity (Figure 5g) and impaired the cellular cannibalism (Figure 5h) detected after XR and ΓδBN+ΞP treatments. Altogether, these results indicate that the NOX5-dependent ROS production is required for the induction of senescence and cellular cannibalism detected after the treatment of cancer cells with XR or GdBN+XR treatments.

### References

1. Schaue D, McBride WH. Opportunities and challenges of radiotherapy for treating cancer. Nat Rev Clin Oncol 2015, 12(9): 527-540.
2. Beasley M, Driver D, Dobbs HJ. Complications of radiotherapy: improving the therapeutic index. Cancer Imaging 2005, 5: 78-84.
3. Hainfeld JF, O'Connor MJ, Dilmanian FA, Slatkin DN, Adams DJ, Smilowitz HM. Micro-CT enables micro localisation and quantification of Her2-targeted gold nanoparticles within tumour regions. Br J Radiol 2011, 84(1002): 526-533.
4. Dorsey JF, Sun L, Job DY, Witztum A, Kao GD, Alonso-Basanta M, et al. Gold nanoparticles in radiation research: potential applications for imaging and radiosensitization. Transl Cancer Res 2013, 2(4): 280-291.
5. Taupin F, Flaender M, Delorme R, Brochard T, Mayol JF, Arnaud J, et al. Gadolinium nanoparticles and contrast agent as radiation sensitizers. Phys Med Biol 2015, 60(11): 4449-4464.
6. McQuaid HN, Muir MF, Taggart LE, McMahon SJ, Coulter JA, Hyland WB, et al. Imaging and radiation effects of gold nanoparticles in tumour cells. Sci Rep 2016, 6: 19442.
7. Zhu J, Zhao L, Cheng Y, Xiong Z, Tang Y, Shen M, et al. Radionuclide (131)I-labeled multifunctional dendrimers for targeted SPECT imaging and radiotherapy of tumors. Nanoscale 2015, 7(43): 18169-18178.
8. Mi Y, Shao Z, Vang J, Kaidar-Person O, Wang AZ. Application of nanotechnology to cancer radiotherapy. Cancer Nanotechnol 2016, 7(1): 11.
9. Le Duc G, Miladi I, Alric C, Mowat P, Brauer-Krisch E, Bouchet A, et al. Toward an image-guided microbeam radiation therapy using gadolinium-based nanoparticles. ACS Nano 2011, 5(12): 9566-9574.
10. Dufort S, Bianchi A, Henry M, Lux F, Le Duc G, Josserand V, et al. Nebulized gadolinium-based nanoparticles: a theranostic approach for lung tumor imaging and radiosensitization. Small 2015, 11(2): 215-221.
11. Hainfeld JF, Slatkin DN, Smilowitz HM. The use of gold nanoparticles to enhance radiotherapy in mice. Phys Med Biol 2004, 49(18): N309-315.
12. Eriksson D, Stigbrand T. Radiation-induced cell death mechanisms. Tumour Biol 2010, 31(4): 363-372.
13. Raza SQ, Martins I, Voisin L, Dakhli H, Paoletti A, Law F, et al. Entescence, a senescence program initiated by cellular cannibalism
14. Martins I, Raza SQ, Voisin L, Dakhli H, Allouch A, Law F, et al. Anticancer chemotherapy and radiotherapy trigger both non-cell-autonomous and cell-autonomous deaths
15. Gire V, Dulic V. Senescence from G2 arrest, revisited. Cell Cycle 2015, 14(3): 297-304.
16. Baus F, Gire V, Fisher D, Piette J, Dulic V. Permanent cell cycle exit in G2 phase after DNA damage in normal human fibroblasts. EMBO J 2003, 22(15): 3992-4002.
17. Lu T, Finkel T. Free radicals and senescence. Exp Cell Res 2008, 314(9): 1918-1922.
18. Zafarullah M, Li WQ, Sylvester J, Ahmad M. Molecular mechanisms of N-acetylcysteine actions. Cell Mol Life Sci 2003, 60(1): 6-20.
19. Lambeth JD. NOX enzymes and the biology of reactive oxygen. Nat Rev Immunol 2004, 4(3): 181-189.
20. Drummond GR, Selemidis S, Griendling KK, Sobey CG. Combating oxidative stress in vascular disease: NADPH oxidases as therapeutic targets. Nat Rev Drug Discov 2011, 10(6): 453-471.
21. Weyemi U, Redon CE, Aziz T, Choudhuri R, Maeda D, Parekh PR, et al. Inactivation of NADPH oxidases NOX4 and NOX5 protects human primary fibroblasts from ionizing radiation-induced DNA damage. Radiat Res 2015, 183(3): 262-270.
22. Gmeiner WH, Ghosh S. Nanotechnology for cancer treatment. Nanotechnol Rev 2015, 3(2): 111-122.
23. Kobayashi K, Usami N, Porcel E, Lacombe S, Le Sech C. Enhancement of radiation effect by heavy elements. Mutat Res 2010, 704(1-3): 123-131.
24. Collado M, Blasco MA, Serrano M. Cellular senescence in cancer and aging. Cell 2007, 130(2): 223-233.
25. Xue W, Zender L, Miething C, Dickins RA, Hernando E, Krizhanovsky V, et al. Senescence and tumour clearance is triggered by p53 restoration in murine liver carcinomas. Nature 2007, 445(7128): 656-660.
26. Ventura A, Kirsch DG, McLaughlin ME, Tuveson DA, Grimm J, Lintault L, et al. Restoration of p53 function leads to tumour regression in vivo. Nature 2007, 445(7128): 661-665.
27. Chen Z, Trotman LC, Shaffer D, Lin HK, Dotan ZA, Niki M, et al. Crucial role of p53-dependent cellular senescence in suppression of Pten-deficient tumorigenesis. Nature 2005, 436(7051): 725-730.
28. Stein GH, Drullinger LF, Soulard A, Dulic V. Differential roles for cyclin-dependent kinase inhibitors p21 and p16 in the mechanisms of senescence and differentiation in human fibroblasts. Mol Cell Biol 1999, 19(3): 2109-2117.
29. Futreal PA, Barrett JC. Failure of senescent cells to phosphorylate the RB protein. Oncogene 1991, 6(7): 1109-1113.
30. Lundberg AS, Hahn WC, Gupta P, Weinberg RA. Genes involved in senescence and immortalization. Curr Opin Cell Biol 2000, 12(6): 705-709.
31. Ewald JA, Desotelle JA, Wilding G, Jarrard DF. Therapy-induced senescence in cancer. J Natl Cancer Inst 2010, 102(20): 1536-1546.
32. Mikkelsen RB, Wardman P. Biological chemistry of reactive oxygen and nitrogen and radiation-induced signal transduction mechanisms. Oncogene 2003, 22(37): 5734-5754.
33. Bedard K, Krause KH. The NOX family of ROS-generating NADPH oxidases: physiology and pathophysiology. Physiol Rev 2007, 87(1): 245-313.
34. Coppe JP, Desprez PY, Krtolica A, Campisi J. The senescence-associated secretory phenotype: the dark side of tumor suppression. Annu Rev Pathol 2010, 5: 99-118.

## Claims

1. A nanoparticle for use in a method of treating a tumor in a subject in need thereof, the method comprising
a. administering an efficient amount of a suspension of nanoparticles to the tumor of a subject in need thereof, said nanoparticles comprising an element with an atomic Z number higher than 40, preferably higher than 50, and having a mean hydrodynamic diameter below 10 nm, preferably below 5 nm, for example between 1 and 5 nm, and,
b. exposing said tumor comprising the nanoparticles to an efficient dose of ionizing radiations,
wherein the combined effect of the ionizing radiations and the nanoparticles induce senescence and/or cellular cannibalism to the irradiated tumor cells.

2. The nanoparticle for use of Claim 1, wherein said tumor is exposed to a dose of ionizing radiations per fraction of at least 3 Gy, and preferably between 3 Gy and 9 Gy, more preferably between 5 and 7 Gy, and the total dose is administered preferably in a maximum of 10 fractions.

3. The nanoparticle for use of Claim 1 or 2, further including a step of determining NOX5 and/or ROCK1 expression level or activity in the tumor, prior to the treatment step.

4. The nanoparticle for use of any one of Claims 1-3, wherein the combined effect of the ionizing radiations and the nanoparticles induces an immune response mediated by NOX5 activity, against the tumor cells.

5. The nanoparticle for use of any one of Claims 1-4, further comprising a step of administering an immunotherapeutic agent prior to, or concomitantly, or after the exposure step to ionizing radiations.

6. The nanoparticle for use of Claim 5, wherein said immunotherapeutic drug is selected among the immune checkpoint inhibitors, such as PD1/PDL1 inhibitors and CTLA4 inhibitors.

7. The nanoparticle for use of any one of Claims 1-6, wherein the tumor to be treated is selected among the tumors that have been shown to be resistant to a chemotherapeutic treatment inducing apoptosis.

8. The nanoparticle for use of any one of Claims 1-7, wherein non-irradiated cells are further killed by cellular cannibalism of neighboring irradiated cells.

9. The nanoparticle for use of any one of Claims 1-8, wherein the number of senescent cells in the tumor cells is increased after the treatment by a factor of at least 10%, 20%, 30%, 40% or at least 50%, as compared to the number of senescent cells induced by the same exposure to ionizing radiations but without the presence of nanoparticles.

10. The nanoparticle for use of any one of Claims 1-9, wherein cellular cannibalism is enhanced by a factor of at least 10%, 20%, 30%, 40% or at least 50%, as compared to cellular cannibalism observe by the same exposure to ionizing radiations but without the presence of nanoparticles.

11. The nanoparticle for use of any one of Claims 1-10, wherein the volume of the tumors exposed to the ionizing radiations is smaller than the total volume of the tumor to be treated, for example at least 10% smaller (in volume), or at least 20% smaller (in volume).

12. The nanoparticle for use of any one of Claims 1-11, wherein the method further enables the treatment of tumors located outside of the region exposed to the ionizing radiations.

13. The nanoparticle for use of any one of Claims 1-12, wherein said nanoparticle comprises a rare earth metal, and preferably gadolinium, as a high-Z element.

14. The nanoparticle for use of any one of Claims 1-13, wherein said nanoparticle comprises chelates of high-Z element, for example chelates of rare earth elements.

15. The nanoparticle for use of Claim 14, wherein said nanoparticle comprises
• polyorganosiloxane,
• chelates covalently bound to said polyorganosiloxane,
• high-Z elements complexed by the chelates.
